# EUROPEAN PATENT SPECIFICATION

(11) **EP 2 566 471 B1**
(45) Date of publication and mention of the grant of the patent: **11.03.2015**
(21) Application number: 11727517.2
(22) Date of filing: 06.05.2011
(51) Int. Cl.: A61K 31/122, A61K 31/593, A61P 19/00

(54) **EFFECTS OF THE ASSOCIATION OF VITAMIN K2 AND VITAMIN D3 IN THE CONSOLIDATION OF A BONE FRACTURE**
EFFEKTE DER ASSOZIATION VON VITAMIN K2 UND VITAMIN D3 BEI DER KONSOLIDIERUNG EINER KNOCHENFRAKTUR
EFFETS DE L'ASSOCIATION DE LA VITAMINE K2 ET DE LA VITAMINE D3 DANS LA CONSOLIDATION D'UNE FRACTURE OSSEUSE

(30) Priority: 07.05.2010 IT MI20100815
(43) Date of publication of application: 13.03.2013
(73) Proprietor: Pharmanutra S.p.A., 56122 Pisa (IT)
(72) Inventor: LACORTE, Andrea, 56014 Pisa (IT); TARANTINO, Germano, 56014 Pisa (IT); BRANDI, Maria, Luisa, 56014 Pisa (IT)
(74) Representative: Hoffmann Eitle
(86) International application number: PCT/IB2011/000968
(87) International publication number: WO 2011/089531

(56) References cited:
- WO-A2-2009/095798
- US-A1- 2008 220 094
- GIGANTE A. ET AL.: "Vitamin K and D association stimulates in vitro osteoblast differentiation of fracture site derived human mesenchymal stem cells", JOURNAL OF BIOLOGICAL REGULATORS AND HOMEOSTATIC AGENTS, WICHTIG EDITORE, MILAN, IT, vol. 22, no. 1, 1 January 2008 (2008-01-01), pages 35-44, XP009140175, ISSN: 0393-974X
- IWAMOTO, J. ET AL.: "Vitamin K2 promotes bone healing in rat femoral osteotomy model with or without glucocorticoid treatment", CALCIF TISSUE INT, vol. 86, March 2010 (2010-03), pages 234-241, XP002606001,
- KNAPEN M H J ET AL: "Vitamin K2 supplementation improves hip bone geometry and bone strength indices in postmenopausal women", OSTEOPOROSIS INTERNATIONAL ; WITH OTHER METABOLIC BONE DISEASES, SPRINGER-VERLAG, LO LNKD- DOI:10.1007/S00198-007-0337-9, vol. 18, no. 7, 8 February 2007 (2007-02-08), pages 963-972, XP019511781, ISSN: 1433-2965
- SCHURGERS ET AL: "Vitamin K2 improves bone strength in postmenopausal women", INTERNATIONAL CONGRESS SERIES, EXCERPTA MEDICA, AMSTERDAM, NL LNKD- DOI:10.1016/J.ICS.2006.08.006, vol. 1297, 1 March 2007 (2007-03-01), pages 179-187, XP022003753, ISSN: 0531-5131
- SHEA, M.K. & BOOTH, S.L.: "Update on the role of vitamin K in skeletal health", NUTRITION REVIEWS, vol. 66, no. 10, 2008, pages 549-557, XP002606002,

## Description

The present invention relates to a composition comprising an association of vitamins for use in treating the consolidation of a bone fracture. In particular, the present invention relates to a composition comprising an association of vitamins for use in treating post-fracture bone regeneration.

Osteoporosis is defined as a skeletal disorder characterized by reduced bone strength, which predisposes an individual to an increased risk of fractures. Bone strength reflects first and foremost the behaviour of two variables: bone density and bone quality. Minimal trauma fractures, a consequence of a reduction in bone strength, are known to be an economic and social problem. While fractures of the femur are at times also associated with mortality in the subjects, other minimal trauma fractures can in any case result in disability.

Distal radius fractures are the most common upper limb fractures, with an incidence of 100 per 100,000 in the 35-44 year age range, increasing to 500 per 100,000 in the 55-75 year age range. The associated risk factors may include poor visual acuity, number of falls, frequent walking and impaired neuromuscular function. Humerus fractures, less common, are associated with severe, prolonged disability. In the 30-49 year age range, the incidence is 3.3 per 100,000 and it increases to 20 per 100,000 in the 50-69 year age group.

The risk factors may include a recent decline in the state of health, insulin-dependent diabetes mellitus, scant motor activity and neuromuscular weakness.

Irrespective of the anatomic site of the fracture, the objectives of consolidation include recovery of the function temporarily lost and recovery of bone biomechanical competence.

Four stages characterize the cascade of fracture consolidation: inflammation, granulation, callus formation and modelling-remodelling.

In the first stage, the inflammatory cells invade the haematoma that has developed as a consequence of the vascular destruction resulting from an event which has provoked the fracture. The inflammatory cells begin to degrade the necrotic tissue. The local stimulation prompts the recruitment of mesenchymal cells. In response to the damaged provoked by the fracture, various cytokines are activated, including TGF beta (transforming growth factor beta), PDGF (platelet-derived growth factor) and bone morphogenetic proteins (BMPs), as well as IL-1 (interleukin-1) and IL-6 (interleukin-6). The duration of the inflammation is estimated to be around 7 days.

In the second stage, the mesenchymal cells differentiate into fibroblasts, chondroblasts and osteoblasts. The development of new blood vessels from other already existing ones, angiogenesis, allows the cells to reach the site of the fracture to form granulation tissue between bone fragments. The recruitment of macrophages and other cytokines during this phase, permits resorption of the haematoma. The duration of granulation is estimated to be around 14 day and leads to the formation of a fibrous callus.

In the third stage, the chondrocytes synthesize cartilage, which is subsequently partially calcified (cartilaginous callus), resorbed and then replaced by bone matrix produced by the osteoblasts, creating a callus which is subsequently mineralized (bone callus). The process of bone callus formation and mineralization may last 4-16 weeks, depending on the fracture location. The mineralized callus is visible on x-rays.

In the fourth stage, the remodelling of the callus consists in the replacement of the primary callus with a secondary callus, characterized by the typical lamellar structure of mature bone. The bone is subject to remodelling over time in order to recover its geometry and biomechanical competences. The duration of this phase ranges from 1 to 4 years.

From the foregoing it is evident that the duration of consolidation of a bone fracture entails long recovery times. It would thus be desirable to be able to reduce the duration of consolidation of a bone fracture so as to be able to reduce, in turn, the recovery time.

Therefore, there remains a need to be able to accelerate the process of consolidation of a bone fracture so as to allow a more rapid recovery of joint function while at the same time maintaining the biomechanical properties of a well-regenerated bone.

In particular, there remains a need to be able to intervene in post-fracture bone regeneration.

A.Gigante et al. discloses that vitamin K1 and vitamin D3 association stimulates *in vitro* the differentiation of human mesenchymal stem cells derived from fracture site toward osteoblasts (Journal of Biological Regulators & homeostatic agents, Vol. 22, no1, 35-44 (2008)).

J. Iwamoto et al. discloses that vitamin K2 promotes bone healing after femoral osteotomy in young mice with or without concomitant glucocorticoid treatment (Calcif. Tissue. Int. 86:234-241 (2010)).

The subject matter of the present invention is a composition comprising vitamin K2 in association with vitamin D₃ for use in treating the consolidation of a bone fracture.

Preferred embodiments of the present invention are set forth in the description that follows in the form of preferred embodiments.

Table 1 shows the results of an *in vivo* study.

In the context of the present invention, the acceleration of healing is defined as a shorter time for consolidating the fracture. In contrast, an improvement in healing is defined as an increase in bone strength generated without an effect on the rate of healing.

The composition of the present invention comprises or may consist of vitamin K2 in association with vitamin D₃ for use in treating the consolidation of a bone fracture. Advantageously, said composition is for use in treating post-fracture bone regeneration.

Therefore, in a preferred embodiment, the composition comprises or may consist of vitamin K2 in association with vitamin D3 for use in treating post-fracture bone regeneration.

In particular, the composition of the present invention is indicated when the bone fracture involves the distal radius.

The subject matter of the present invention also relates to the use of vitamin K2 in association with vitamin D₃ to prepare a pharmaceutical, supplement or food composition for treating the consolidation of a bone fracture or for treating post-fracture bone regeneration, in particular of the distal radius.

Vitamin K2 comprises or may consist of the compound menaquinone-7 (MK-7) and/or the compound menaquinone-6 (MK-6), whereas vitamin D3 is cholecalciferol.

In a preferred embodiment, said composition comprises or may consist of the compound menaquinone-7 (MK-7) and/or the compound menaquinone-6 (MK-6) in association with vitamin D₃, cholecalciferol.

In another preferred embodiment, said composition comprises vitamin K2 and vitamin D3 in a ratio by weight comprised from 5:1 to 20:1, preferably 10:1.

In another preferred embodiment, said composition comprises or may consist of the compound menaquinone-7 (MK-7) and of the compound menaquinone-6 (MK-6) in a ratio by weight comprised from 150:1 to 30:1, preferably from 100:1 to 50:1.

The composition of the present invention is in a pharmaceutical form suitable for oral administration. For example, it can be in solid, granular, powder or lyophilized form. Preferably, the composition is in the form of an oral tablet or pill.

The composition of the present invention may be indicated for pharmaceutical, dietetic, nutraceutical or nutritional use.

The Applicant has found that the compound menaquinone-7 (MK-7) and the compound menaquinone-6 (MK-6) assure a high degree of mineralization of fractured bone, whereas vitamin D3 is useful for the intestinal absorption of calcium, which is fixed to the bone.

The composition of the present invention has valid application in the treatment of a fracture of the distal radius.

The composition of the present invention can be administered for a period comprised from 4 to 16 weeks, preferably from 8 to 12 weeks. For example, a composition is administered in the form of one capsule per day for 8 weeks. The composition may comprise vitamin K2 (menaquinone-7 and/or menaquinone-6) and vitamin D3 (cholecalciferol) in a ratio by weight of 9:1, for example 45 micrograms of vitamin K₂ and 5 micrograms of vitamin D₃.

### In vivo Study

In the clinical study, all aspects of bone healing were taken into consideration. Fracture of the distal radius without ulnar styloid fracture (classification AO2) was considered as the experimental model of reference since it entails fewer prejudices tied to the subjectivity of the patient observed. In fact, among the fractures least subject to differences in working load, the radiographic course of healing is easily observable. Whereas ulnar styloid fracture was excluded as it influences the functional state and may represent a prejudice in the observation of the course of fracture consolidation.

The Müller and Coll method of fracture classification with the AO scale (1990) was taken as reference, using both X-rays (radiography) and CAT.

In general, fractures of the distal radius consolidate in 6 weeks with a return to normal functionality and the cortical construction can be shown by radiography in 8 weeks.

Since all immobilization interventions which envisage the use of metal devices interfere with x-ray imaging, an uninvasive or non-invasive treatment (plaster cast) was used to immobilize the fractured limb.

The objective of the present clinical study was to evaluate radiographic consolidation of the fracture of the distal radius at 8 weeks, following daily administration of a composition, in the form of a capsule, comprising vitamin K2 (menaquinone-7 and menaquinone-6) and vitamin D3 (cholecalciferol) in a ratio by weight of 9:1, for example 45 micrograms of vitamin K₂ and 5 micrograms of vitamin D₃ (tested composition).

The x-rays scheduled throughout the study provided objective evidence on the process of fracture consolidation and provided useful information about the rate of consolidation in the treated group versus the control group.

In practical terms, 22 patients were recruited over a 6-month time period; they were divided into two groups. A first treatment group (group 1) containing 10 patients, indicated as T1-T10 and a second control group (group 2) containing 12 patients, indicated as C1-C12.

All patients had an extra-articular wrist fracture (distal radius without ulnar styloid fracture - classification A02). The fractures were immobilized in the emergency room with a uninvasive treatment (plaster cast).

The subjects were recruited within the 10th day after the occurrence of the fracture and the first dose was administered starting from the 10th day.

The use of non-steroidal anti-inflammatory drugs (NSAIDs, including COX-2 inhibitors) was not admitted, whereas doses of aspirin for the prophylaxis of coronary disease (less than 500 mg/day) were considered acceptable.

The patients undergoing treatment were examined after 8 weeks and the x-ray images were collected. Since these fractures consolidate rapidly, week 8 after the occurrence of the fracture coincided with the phase of callus formation.

The effect of the treatment was evaluated using the chisquare test. In practical terms, the percentage difference between group 1 and group 2 was evaluated, with an evaluation of the standard deviation and a 95% confidence interval.

The fracture consolidation profiles at 8 weeks are reported in Table 1.

### Results

In general, a complete consolidation was observed in all 4 profiles in 13 out of 22 patients (T1, C1, C2, T3, T4, C3, T5, T6, T7, T8, T10, C7 and C12 in Table 1), where the exceptions are represented by particularly displaced fractures (T2, T9 and C8 in Table 1).

Between group 1 (treated) and the group 2 (control) a certain difference can be observed, not so much in the number of sides consolidated at 2 months, but rather in the highly transparent gap, which seems to close to a greater extent or more rapidly in the treated patients.

This means that, advantageously, the composition of the present invention is capable of accelerating the calcification of the gap (the fracture gap is the fissure which separates the two bone fragments) as well as accelerating the peripheral repair process.

Moreover, in patients for whom a follow-up x-ray at 4 weeks (total of 4) is available, or those who in the care of hand surgeons due to the type of fracture, a difference was observed in the bone callus, more visible in treated cases. This means that, advantageously, the composition of the present invention is capable of intervening in the repair process, which is rapidly conditioned (4 weeks). Furthermore, the composition of the present invention is capable of intervening in reducing the process of pseudoarthrosis. Pseudoarthrosis is a fracture in which the bone callus does not undergo mineralization and the formation of "real" bone tissue.

**Table 1**

| Patients | | No. of Consolidation Profiles at 8 weeks |
|---|---|---|
| Group 1 (treated) | Group 2 (control) | |
| T1 | | 4/4 perfectly consolidated |
| | C1 | 4/4; gap visible |
| T2 | | 3/4; consolidated; displaced fracture |
| | C2 | 4/4; well consolidated |
| T3 | | 4/4; well consolidated |
| T4 | | 4/4, perfectly consolidated |
| | C3 | 4/4; consolidated |
| T5 | | 4/4; disappearance of gap |
| T6 | | 4/4; well consolidated |
| T7 | | 4/4; perfectly consolidated |
| T8 | | 4/4; consolidated |
| T9 | | 3/4; consolidated; displaced fracture |
| T10 | | 4/4; perfectly consolidated |
| | C4 | 3/4; > resorption of highly transparent gaps |
| | C5 | 3/4; consolidated |
| | C6 | 2/4; gap visible |
| | C7 | 4/4; well consolidated |
| | C8 | 3/4; fracture gap clearly visible; displaced fracture |
| | C9 | 2/4; enlargement of fracture fragments |
| | C10 | 3/4; still displaced |
| | C11 | 2/4; still displaced |
| | C12 | 4/4; well consolidated |

## Claims

1. A composition comprising vitamin K2 in association with vitamin D₃ for use in treating the consolidation of the bone fractures.

2. The composition for use in accordance with claim 1, wherein the treatment is aimed at post-fracture bone regeneration.

3. The composition for use in accordance with claim 1 or 2, wherein the bone fracture is of the distal radius.

4. The composition for use in accordance with any one of claims 1-3, wherein the vitamin K2 comprises the compound menaquinone-7 (MK-7) and/or the compound menaquinone-6 (MK6).

5. The composition for use in accordance with claim 4, wherein said composition comprises the compound menaquinone-7 (MK-7) and the compound menaquinone-6 (MK-6) in a ratio by weight comprised from 150:1 to 30:1, preferably from 100:1 to 50:1.

6. The composition for use in accordance with claim 4 or 5, wherein the compound menaquinone-7 (MK-7) and/or the compound menaquinone-6 (MK-6) are in association with vitamin D3.

7. The composition for use in accordance with any one of claims 1-6, wherein said composition comprises vitamin K2 and vitamin D3 in a ratio by weight comprised from 5:1 to 20:1, preferably 10:1.

8. The composition for use in accordance with any one of claims 1-7, wherein said composition is in a pharmaceutical form suitable for oral administration, preferably in solid, granular, powder or lyophilized form.

9. The composition for use in accordance with any one of claims 1-8, wherein said composition is for pharmaceutical, dietetic, nutraceutic or nutritional use.

10. The composition for use in accordance with any one of claims 1-9, wherein said composition comprising vitamin K2 which comprises menaquinone-7 and/or menaquinone-6, and vitamin D3 in a ratio by weight of 9:1, is administered for a period of time comprised from 4 to 16 weeks, preferably in the form of one capsule per day for 8 weeks.

## Patentansprüche

1. Zusammensetzung, umfassend Vitamin K2 zusammen mit Vitamin D₃, zur Anwendung bei der Behandlung der Konsolidierung von Knochenfrakturen.

2. Zusammensetzung zur Anwendung gemäß Anspruch 1, wobei die Behandlung auf Knochenregeneration nach der Fraktur gerichtet ist.

3. Zusammensetzung zur Anwendung gemäß Anspruch 1 oder 2, wobei die Knochenfraktur eine Fraktur des distalen Radius ist.

4. Zusammensetzung zur Anwendung gemäß einem der Ansprüche 1 bis 3, wobei das Vitamin K2 die Verbindung Menachinon-7 (MK-7) und/oder die Verbindung Menachinon-6 (MK-6) umfasst.

5. Zusammensetzung zur Anwendung gemäß Anspruch 4, wobei die genannte Zusammensetzung die Verbindung Menachinon-7 (MK-7) und die Verbindung Menachinon-6 (MK-6) in einem Gewichtsverhältnis von 150:1 bis 30:1, bevorzugt 100:1 bis 50:1, umfasst.

6. Zusammensetzung zur Anwendung gemäß Anspruch 4 oder 5, wobei die Verbindung Menachinon-7 (MK-7) und/oder die Verbindung Menachinon-6 (MK-6) zusammen mit Vitamin D3 vorliegen.

7. Zusammensetzung zur Anwendung gemäß einem der Ansprüche 1 bis 6, wobei die genannte Zusammensetzung Vitamin K2 und Vitamin D3 in einem Gewichtsverhältnis von 5:1 bis 20:1, bevorzugt 10:1, umfasst.

8. Zusammensetzung zur Anwendung gemäß einem der Ansprüche 1 bis 7, wobei die genannte Zusammensetzung in einer pharmazeutischen Form vorliegt, die zur oralen Verabreichung geeignet ist, bevorzugt in fester, granulärer, pulverförmiger oder lyophilisierter Form.

9. Zusammensetzung zur Anwendung gemäß einem der Ansprüche 1 bis 8, wobei die genannte Zusammensetzung zur pharmazeutischen Anwendung, diätischen Anwendung, nutrazeutischen Anwendung oder zur Ernährungsanwendung bestimmt ist.

10. Zusammensetzung zur Anwendung gemäß einem der Ansprüche 1 bis 9, wobei die genannte Zusammensetzung, umfassend Vitamin K2, welches Menachinon-7 und/oder Menachinon-6 umfasst, und Vitamin D3 in einem Gewichtsverhältnis von 9:1, über einen Zeitraum von 4 bis 16 Wochen, bevorzugt in der Form einer Kapsel pro Tag über 8 Wochen, verabreicht wird.

## Revendications

1. Composition comprenant de la vitamine K2 en association avec de la vitamine D₃ pour une utilisation dans le traitement de la consolidation des fractures osseuses.

2. Composition pour une utilisation selon la revendication 1, dans laquelle le traitement est destiné à une régénération osseuse après fracture.

3. Composition pour une utilisation selon la revendication 1 ou 2, dans laquelle la fracture osseuse est une fracture du radius distal.

4. Composition pour une utilisation selon l'une quelconque des revendications 1 à 3, dans laquelle la vitamine K2 comprend le composé ménaquinone-7 (MK-7) et/ou le composé ménaquinone-6 (MK6).

5. Composition pour une utilisation selon la revendication 4, dans laquelle ladite composition comprend le composé ménaquinone-7 (MK-7) et le composé ménaquinone-6 (MK-6) dans un rapport en poids compris entre 150:1 et 30:1, de préférence entre 100:1 et 50:1.

6. Composition pour une utilisation selon la revendication 4 ou 5, dans laquelle le composé ménaquinone-7 (MK-7) et/ou le composé ménaquinone-6 (MK-6) est/sont en association avec la vitamine D3.

7. Composition pour une utilisation selon l'une quelconque des revendications 1 à 6, dans laquelle ladite composition comprend de la vitamine K2 et de la vitamine D3 dans un rapport en poids compris entre 5:1 et 20:1, de préférence 10:1.

8. Composition pour une utilisation selon l'une quelconque des revendications 1 à 7, dans laquelle ladite composition est sous une forme pharmaceutique appropriée pour une administration par voie orale, de préférence sous une forme solide, sous une forme granulaire, sous une forme de poudre ou sous une forme lyophilisée.

9. Composition pour une utilisation selon l'une quelconque des revendications 1 à 8, dans laquelle ladite composition est destinée à une utilisation pharmaceutique, diététique, nutraceutique ou nutritionnelle.

10. Composition pour une utilisation selon l'une quelconque des revendications 1 à 9, dans laquelle ladite composition comprenant de la vitamine K2 qui comprend de la ménaquinone-7 et/ou de la ménaquinone-6, et de la vitamine D3 dans un rapport en poids de 9:1, est administrée pendant une durée comprise entre 4 et 16 semaines, de préférence sous forme d'une capsule par jour pendant 8 semaines.
